# EUROPÄISCHE PATENTSCHRIFT

(11) **EP 1 404 238 B1**
(45) Veröffentlichungstag und Bekanntmachung des Hinweises auf die Patenterteilung: **09.05.2007**
(21) Anmeldenummer: 03709537.9
(22) Anmeldetag: 10.04.2003
(51) Int. Cl.: A61B 17/28

(54) **VORRICHTUNG ZUM VERBINDEN EINER LÖSBAR AN EINEM GEHÄUSE ANGEORDNETEN FUNKTIONSEINHEIT**
DEVICE FOR CONNECTING A FUNCTIONAL UNIT WHICH CAN BE DETACHABLY ARRANGED ON A HOUSING
DISPOSITIF POUR RELIER UNE UNITE FONCTIONNELLE MISE EN PLACE DE FA ON AMOVIBLE CONTRE UN BOITIER

(30) Priorität: 16.04.2002 CH 634022002; 02.12.2002 CH 203102
(43) Veröffentlichungstag der Anmeldung: 07.04.2004
(73) Patentinhaber: Alcon Grieshaber AG, 8203 Schaffhausen (CH)
(72) Erfinder: ATTINGER, Jürg, CH-8260 Stein am Rhein (CH); MAAG, Werner, CH-8750 Glarus (CH); EGLI, Lorenz, CH-8212 Neuhausen am Rheinfall (CH)
(74) Vertreter: Spierenburg, Pieter
(86) Internationale Anmeldenummer: PCT/CH2003/000234
(87) Internationale Veröffentlichungsnummer: WO 2003/086210

(56) Entgegenhaltungen:
- EP-A- 0 705 571
- DE-A- 4 440 546
- DE-C- 19 851 268
- US-A- 4 258 716
- US-A1- 2001 056 286

## Beschreibung

Die Erfindung bezieht sich auf eine Vorrichtung zum lösbaren Verbinden zwei als Gehäuse ausgebildeter Baueinheiten eines am distalen Ende mit einem zur Durchführung ophthalmologischer Eingriffe motorisch oder manuell betätigbaren Werkzeug versehenen Instruments, bei welchem die beiden jeweils zur Lagerung eines Schubgliedes ausgebildeten Gehäuse mittels eines entlang einer nut- oder schlitzförmigen Führungsbahn gleitenden Rasterzapfens axial zusammenschiebbar sowie relativ zueinander um eine gemeinsame Längsachse drehbar und in der Endstellung infolge des in eine Ausnehmung eingreifenden Rasterzapfens formschlüssig miteinander verbunden sind.

Aus der DE 44 40 546 A ist eine Einrichtung zur Durchführung laparoskopischer Eingriffe, insbesondere zur Untersuchung, Betrachtung und Behandlung von Bauchhöhlen bekannt, welche mehrere auswechselbar in einem Magazin angeordnete und jeweils mit einem Werkzeug versehene Instrumentenköpfe, ein mit einem röhrchenförmigen Schaft und einem Handgriff versehenes Halteteil sowie eine erste Kupplung und eine zweite Kupplung zum formschlüssigen Verbinden der einzelnen Elemente umfasst, wobei die erste Kupplung einen in jedem Instrumentenkopf angeordneten und etwa L-förmig ausgebildeten Führungsschlitz zum Einführen eines in dem röhrchenförmigen Schaft angeordneten Bolzens aufweist und die zweite Kupplung mit einem an einer ersten Schubstange angeordneten Quersteg sowie mit mehreren an einer zweiten Schubstange angeordneten Führungsstegen und mit einer gabelförmigen Klaue versehen ist. Bei dieser Einrichtung ist eine exakte Verbindung des lanzenförmigen Schafts mit dem jeweiligen Instrumentenkopf nicht gewährleistet und eine taktile Einrastung durch den Chirurgen nicht feststellbar. Zudem sind die einzelnen Elemente der bei dieser Einrichtung zusätzlich vorgesehenen zweiten Kupplung in der Herstellung und Montage relativ aufwendig und kostenintensiv.

Die Druckschrift US 2001/056286 offenbart ein Instrument zur Durchführung ophthalmologischer Eingriffe, welches ein als Handgriff ausgebildetes erstes Gehäuse mit zwei länglichen Gehäuseteilen, einen dazwischen angeordneten und in Längsrichtung orientierten Tragarm sowie eine mit einem hülsenförmigen zweiten Gehäuse (91) versehene Funktionseinheit mit einem am distalen Ende angeordneten Werkzeug umfasst, welches mittels jeweils in den beiden Gehäusen gelagerter und in axialer Richtung orientierter Schubglieder betätigbar ist. Bei diesem chirurgischen Instrument ist das mit dem Werkzeug versehene zweite Gehäuse mit einer daran angeordneten Überwurfmutter auf ein mit einem Aussengewinde versehenes Kopfstück des ersten Gehäuses derart aufschraubbar, dass die beiden Schubglieder zur Betätigung des Werkzeuges miteinander wirkverbunden und in axialer Richtung verschiebbar sind.

Aus der US-A 4,258,716 ist ein weiteres Instrument zur Durchführung ophthalmologischer Eingriffe bekannt, welches ein als Handgriff ausgebildetes erstes Gehäuse sowie eine mit einem hülsenförmigen zweiten Gehäuse versehene Funktionseinheit umfasst, wobei das erste Gehäuse einen daran gelagerten Hebel sowie ein Kopfstück und das zweite Gehäuse eine daran angeformte röhrchenförmige Sonde mit am distalen Ende angeordneten Schneidelementen umfasst. Bei diesem Instrument sind die beiden Gehäuse durch eine aufschraubbare Überwurfmutter derart miteinander verbunden, dass ein an dem Hebel angeformter Mitnehmer mit einem in dem zweiten Gehäuse gelagerten Schubglied zusammenwirkt und durch eine um die Längsachse orientierte Drehbewegung der Sonde die Schneidelemente betätigbar sind.

Der Erfindung liegt die Aufgabe zugrunde, eine Vorrichtung gemäss der eingangs genannten Art dahingehend zu verbessern, dass unter Beibehaltung einer sicheren und sterilen Handhabung die beiden in den Händen der Bedienungsperson (Chirurgen) gehaltenen Gehäuse in axialer Richtung zusammenschiebbar und möglichst schnell formschlüssig miteinander verbindbar sind und dabei gleichzeitig die in axialer Richtung jeweils in den Gehäusen gelagerten Schubglieder ohne zusätzliche Hilfsmittel kraftschlüssig miteinander wirkverbunden sind.

Die vorstehende Aufgabe wird erfindungsgemäss durch die im kennzeichnenden Teil des Anspruchs 1 angegebenen Merkmale gelöst, wobei weitere Merkmale und Ausgestaltungen der Vorrichtung jeweils in den Unteransprüchen angegeben sind.

Mit der erfindungsgemässen Vorrichtung wird es dem Chirurgen ermöglicht, ein als Handgriff ausgebildetes erstes Gehäuse mit einem mit dem Werkzeug versehenen zweiten Gehäuse relativ einfach lösbar miteinander zu verbinden, wobei bei der um die Längsachse der beiden Gehäuse orientierten Drehbewegung die definitive formschlüssige Verbindung und somit eine sichere Funktionsfähigkeit des ophthalmologischen Instruments von dem Chirurgen taktil wahrnehmbar ist.

Ein weiterer Vorteil der erfindungsgemässen Vorrichtung besteht darin, dass in zusammengebautem Zustand des ersten Gehäuses mit dem zweiten Gehäuse die beiden Schubglieder für die Betätigung des Werkzeuges ohne zusätzliche kuppelnde Hilfsmittel miteinander wirkverbunden sind und somit die Vorrichtung relativ wenig Funktionselemente aufweist und wirtschaftlich herstellbar ist.

Nachstehend werden unter Bezugnahme auf die Zeichnung mehrere Ausführungsbeispiele der erfindungsgemässen Vorrichtung beschrieben. Es zeigt:
- **Fig. 1**: ein erstes Ausführungsbeispiel eines räumlich dargestellten chirurgischen Instruments mit einem als Handgriff ausgebildeten ersten Gehäuse für eine austauschbar daran angeordnete Funktionseinheit;
- **Fig.2**: das in Ansicht dargestellte Instrument gemäss Fig.1 mit der relativ zu dem Gehäuse in demontierter Stellung dargestellten Funktionseinheit;
- **Fig.3**: das in grösserem Massstab und im Schnitt dargestellte vordere Teilstück des Instruments gemäss Fig.1 oder Fig.2 mit der teilweise an einem Verbindungsglied angeordneten Funktionseinheit;
- **Fig.4**: ein zweites Ausführungsbeispiel des Instruments mit der in demontierter Stellung dargestellten Funktionseinheit;
- **Fig.5**: eine erste Variante des teilweise im Schnitt dargestellten Verbindungsgliedes für das Instrument gemäss Fig.4;
- **Fig.6**: das in Seitenansicht dargestellte Verbindungsglied gemäss Fig.5;
- **Fig.7**: ein teilweise im Schnitt dargestelltes Ausführungsbeispiel des Instruments gemäss Fig.4 mit der in demontierter Stellung dargestellten Funktionseinheit;
- **Fig.8**: eine zweite Variante des teilweise im Schnitt dargestellten Verbindungsgliedes für das Instrument gemäss Fig.7;
- **Fig.9**: das in Seitenansicht dargestellte Verbindungsglied gemäss Fig. 8;
- **Fig.10**: ein in räumlicher Ansicht dargestelltes drittes Ausführungsbeispiel des Instruments mit daran angeordneter Funktionseinheit;
- **Fig.11**: das in Ansicht dargestellte Instrument gemäss Fig.10 mit der in demontierter Stellung dargestellten Funktionseinheit;
- **Fig.12**: das teilweise im Schnitt sowie in grösserem Massstab dargestellte vordere Teilstück des Instruments gemäss Fig.10 und Fig.11 mit der an dem Verbindungsglied angeordneten Funktionseinheit;
- **Fig.13**: ein teilweise im Schnitt dargestelltes Verbindungsglied für das Instrument gemäss der Figuren 10 bis 12;
- **Fig.14**: das gemäss der in Fig.13 eingezeichneten Linie A-A im Schnitt dargestellte Verbindungsglied;
- **Fig.15**: ein in räumlicher Ansicht dargestelltes viertes Ausführungsbeispiel des Instruments mit daran angeordneter Funktionseinheit;
- **Fig.16**: ein im Schnitt sowie in grösserem Massstab dargestelltes Teilstück des Instruments gemäss Fig.15 mit der in demontierter Stellung dargestellten Funktionseinheit;
- **Fig.17**: die gemäss der in Fig. 16 eingezeichneten Linie B-B im Schnitt dargestellte Funktionseinheit;
- **Fig.18**: ein fünftes Ausführungsbeispiel des teilweise dargestellten Instruments mit der in demontierter Stellung dargestellten Funktionseinheit;
- **Fig.19**: ein weiteres Ausführungsbeispiel des in demontierter Stellung dargestellten und mit dem Gehäuse, einem Zwischenstück und der Funktionseinheit versehenen Instruments;
- **Fig.20**: ein erstes Anwendungsbeispiel der mit einem eingeschraubten Zwischenstück versehenen Funktionseinheit für das Instrument gemäss Fig.19;
- **Fig.21**: ein zweites Anwendungsbeispiel mit dem auf das Verbindungsglied aufgeschobenen Zwischenstück für die lösbare Verbindung der Funktionseinheit an dem Gehäuse;
- **Fig.22**: das Instrument gemäss Fig.19 mit den einzelnen in zusammengebautem Zustand dargestellten Elementen;
- **Fig.23**: ein Teilstück des räumlich dargestellten Verbindungsgliedes für das jeweilige Instrument gemäss der Figuren 1 bis 22;
- **Fig.24**: das in Ansicht dargestellte Verbindungsglied gemäss Fig.23 mit daran angeordneter Führungsbahn; und
- **Fig.25**: die in grösserem Massstab sowie als Abwicklung dargestellte Führungsbahn des Verbindungsgliedes.

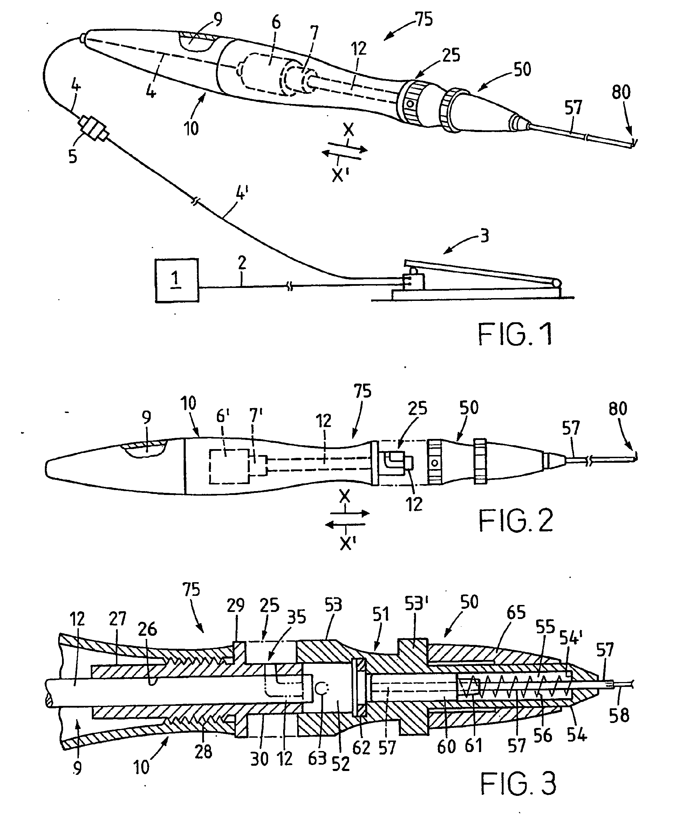

Zur allgemeinen Verdeutlichung der Erfindung sind in den einzelnen Figuren unterschiedlich ausgebildete Instrumente dargestellt, wobei jedes Instrument im wesentlichen ein als Handgriff ausgebildetes Gehäuse 10 sowie eine Funktionseinheit 50 umfasst. In dem Gehäuse 10 ist eine erste Stange 12 angeordnet, welche zur Erreichung einer in axialer Richtung orientierten Bewegung entweder mit motorisch oder manuell betätigbaren Mitteln wirkverbunden ist. Mittels der ersten Stange 12 und eines damit wirkverbundenen Schiebebolzens 60 wird eine in der Funktionseinheit 50 angeordnete röhrchenförmige Sonde 57 relativ zu einer koaxial darin angeordneten zweiten Stange 58 (Fig.3) in axialer Richtung verschoben. Infolge der axialen Relativbewegung der Sonde in Bezug auf die zweite Stange 58 kann ein am distalen Ende derselben angeordnetes Werkzeug 80 betätigt werden.

An dieser Stelle wird darauf hingewiesen, dass die in den einzelnen Figuren schematisch dargestellten Schneid-, Greif- oder Klemmelemente nicht Gegenstand dieser Erfindung sind und nachstehend generell als Werkzeug 80 bezeichnet werden. Die in den einzelnen Figuren montiert oder demontiert dargestellten Funktionseinheiten 50 sind analog der nachstehend in Verbindung mit Fig.3 beschriebenen Funktionseinheit 50 ausgebildet.

Fig.1 zeigt als erstes Ausführungsbeispiel ein räumlich dargestelltes und in der Gesamtheit mit 75 bezeichnetes chirurgisches Instrument. Das Instrument 75 ist zur Durchführung ophthalmologischer Eingriffe ausgebildet und umfasst ein als Handgriff ausgebildetes Gehäuse 10, ein daran angeordnetes Verbindungsglied 25 sowie die auswechselbar daran angeordnete Funktionseinheit 50. Am vorderen Ende der Funktionseinheit 50 ist die als Hohlnadel ausgebildete Sonde 57 angeordnet, in welcher die am distalen Ende mit dem Werkzeug 80 versehene zweite Stange 58 (Fig.3) gelagert ist.

Bei dem in Fig.1 dargestellten ersten Ausführungsbeispiel ist zur Betätigung des Werkzeuges 80 im Innenraum 9 des Gehäuses 10 ein Antrieb 6 angeordnet. Der Antrieb 6 ist an dem einen Ende über eine Kupplung 7 mit der in axialer Richtung gemäss Pfeilrichtung X oder X' verschiebbaren ersten Stange 12 wirkverbunden. An dem anderen Ende ist der Antrieb 6 über eine Leitung, beispielsweise über eine erste Leitung 4 und 4' unter Zwischenschaltung eines Kupplungsstücks 5 an einen Schalter 3 angeschlossen. Der beispielsweise über ein Pedal oder dergleichen mit dem Fuss betätigbare Schalter 3 ist über eine zweite Leitung 2 an eine Energiequelle 1 angeschlossen.

Die in dem-Gehäuse 10 angeordnete und mit dem Antrieb 6 wirkverbundene erste Stange 12 kann von dem Antrieb 6 entweder pneumatisch, hydraulisch oder elektromotorisch in axialer Richtung bewegt werden. Bei einer ersten Variante ist der Antrieb 6 für die pneumatische Betätigung mit einer als Druckerzeuger ausgebildeten Energiequelle 1 wirkverbunden. Bei einer zweiten Variante ist der Antrieb als elektromotorischer Antrieb 6 ausgebildet und zur Betätigung desselben an eine elektrische Energiequelle 1 angeschlossen. Bei einer weiteren Variante ist der Antrieb als hydraulisch betätigbarer Antrieb 6 ausgebildet und zur Betätigung desselben mit einer als hydraulischer Druckerzeuger ausgebildeten Energiequelle 1 wirkverbunden. Die einzelnen Antriebsvarianten sind vorzugsweise mittels des mit dem Fuss betätigbaren Schalters 3 aktivierbar.

Fig.2 zeigt als erste Variante das in Ansicht dargestellte Instrument 75 und man erkennt das Gehäuse 10 mit dem daran angeordneten Verbindungsglied 25 sowie die relativ dazu im Abstand (demontiert) dargestellte Funktionseinheit 50. Abweichend von dem Instrument 75 gemäss Fig.1 ist bei dieser Variante in dem Innenraum 9 des Gehäuses 10 beispielsweise eine Batterie 6' auswechselbar angeordnet. Die Batterie 6' ist über eine Kupplung 7' mit der gemäss Pfeilrichtung X oder X' in axialer Richtung verschiebbaren ersten Stange 12 in nicht näher dargestellter Weise wirkverbunden.

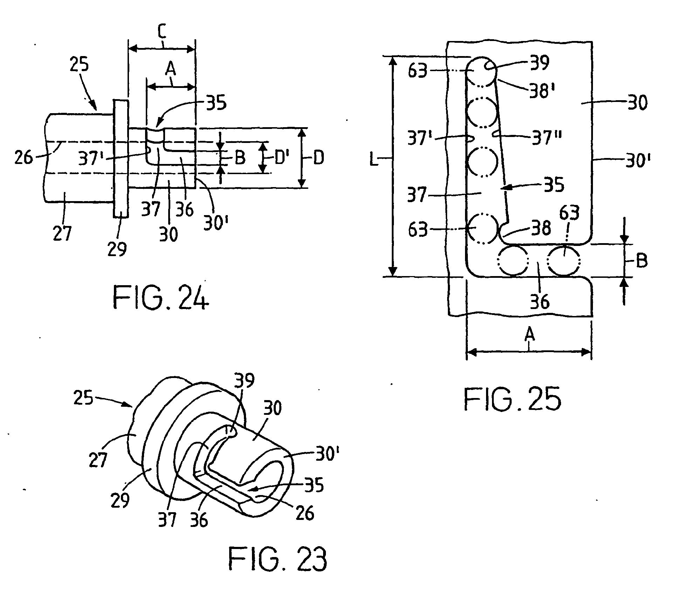

Fig.3 zeigt das in grösserem Massstab sowie im Schnitt dargestellte vordere Teilstück des Instruments 75 gemäss Fig.1 oder Fig.2 und man erkennt ein Teilstück des Gehäuses 10 sowie die teilweise demontiert dargestellte Funktionseinheit 50. In dem Innenraum 9 des Gehäuses 10 ist das Verbindungsglied 25 mit der koaxial darin gelagerten ersten Stange 12 angeordnet. Das mit in axialer Richtung orientierter Durchgangsbohrung 26 für die erste Stange 12 versehene Verbindungsglied 25 hat eine teilweise mit einem Gewinde 28 versehene Führungshülse 27, einen daran angeordneten Flansch 29 sowie ein Anschlussteil 30. Das Verbindungsglied 25 ist mit dem Gewindeteilstück 28 in das am vorderen Ende mit einem nicht näher bezeichneten Innengewinde versehenen Gehäuse 10 eingeschraubt. Das am Flansch 29 angeformte zylindrische Anschlussteil 30 ist mit einer Führungsbahn 35 zum Einführen und formschlüssigen Verbinden eines an der Funktionseinheit 50 angeordneten Rasterzapfens 63 versehen. Die zum Einführen des Rasterzapfens 63 ausgebildete Führungsbahn 35 wird später in Verbindung mit den Figuren 23 bis 25 beschrieben.

Die in Fig.3 im Schnitt dargestellte Funktionseinheit 50 umfasst ein mit angeformten Griffteilen 53 und 53' versehenes Gehäuse 51 sowie eine daran angeformte und in axialer Richtung orientierte Führungshülse 54. An der Führungshülse 54 ist vorzugsweise eine Haube 65 angeordnet und mit nicht dargestellten Mitteln befestigt. Das Gehäuse 51 hat eine zum Aufschieben auf das Anschlussteil 30 ausgebildete erste Ausnehmung 52 mit darin angeordneter Dichtung 62 sowie eine zweite Ausnehmung 55. In der zweiten Ausnehmung 55 ist der in nicht dargestellter Weise mit der röhrchenförmigen Sonde 57 wirkverbundene Schiebebolzen 60 sowie eine Druckfeder 56 angeordnet. Die an dem vorderen Ende mit dem Werkzeug 80 (Fig.1,2) versehene zweite Stange 58 ist in nicht dargestellter Weise derart in dem Gehäuse 51 fixiert, dass der Schiebebolzen 60 zusammen mit der röhrchenförmigen Sonde 57 zur Betätigung des Werkzeuges 80 relativ zu der zweiten Stange 58 in axialer Richtung verschiebbar ist. Die Druckfeder 56 ist mit dem einen Ende an einem Absatz 61 des Schiebebolzens 60 angeordnet und mit dem anderen Ende an der Innenwand 54' der Führungshülse 54 abgestützt.

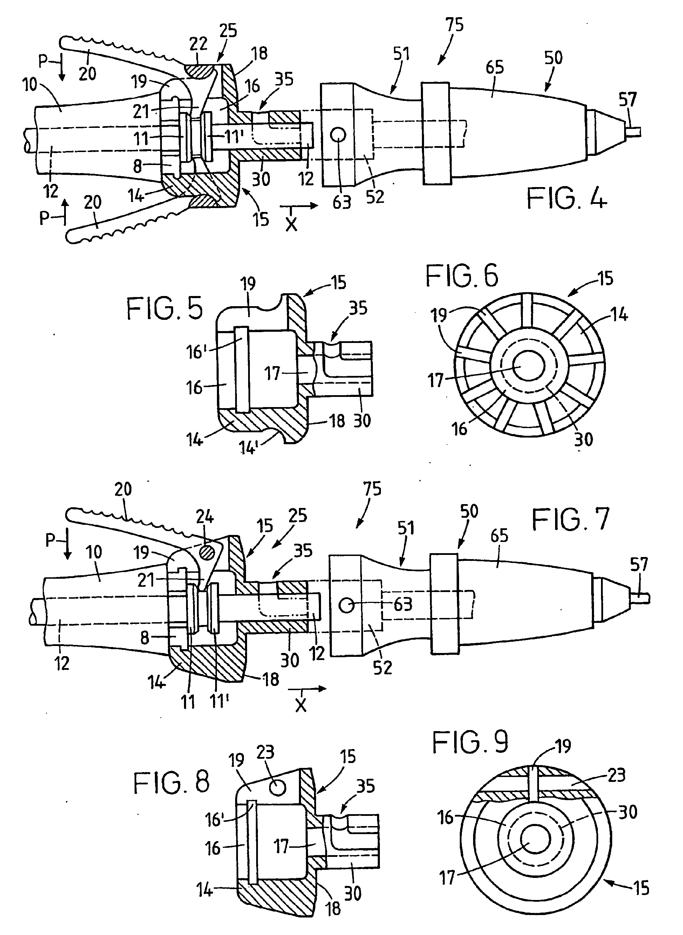

Fig.4 zeigt als zweites Ausführungsbeispiel ein manuell betätigbares Instrument 75 und man erkennt ein Teilstück des Gehäuses 10 mit dem daran angeordneten Verbindungsglied 25 sowie die demontiert dargestellte Funktionseinheit 50. Abweichend.von dem Instrument 75 gemäss Fig.1 und Fig.2 ist das am vorderen Ende des Gehäuses 10 angeordnete Verbindungsglied 25 mit einem Kopfstück 15 versehen. An dem Kopfstück 15 ist das mit zwei oder mehreren in Umfangsrichtung verteilten Schnappgliedern 8 versehene Gehäuse 10 befestigt. Weiterhin sind an dem Kopfstück 15 mindestens zwei durch einen Ring 22 oder dergleichen gehaltene Hebel 20 gelagert, welche jeweils mit einem Mitnehmer 21 mit der ersten Stange 12 wirkverbunden sind. Der im Profilquerschnitt etwa halbkreisförmig ausgebildete Ring 22 ist beispielsweise aus elastischem Werkstoff hergestellt. Im dargestellten Ausführungsbeispiel sind die beiden Hebel 20 mit den Mitnehmern 21 zwischen zwei axial im Abstand zueinander an der ersten Stange 12 angeordneten zirkulären Halterungen 11 und 11' angeordnet. Bei Betätigung der beiden Hebel 20 gemäss Pfeilrichtung P wird die erste Stange 12 gemäss Pfeilrichtung X in axialer Richtung verschoben. Die in axialer Richtung orientierte Bewegung der ersten Stange 12 bewirkt, dass diese bei montierter Funktionseinheit 50 mit dem Schiebebolzen 60 (Fig.3) in Eingriff gebracht und infolge davon das Werkzeug 80 (Fig.1,2) betätigt wird.

In Fig.5 und Fig.6 ist das Kopfstück 15 für das Verbindungsglied 25 gemäss Fig.4 im Schnitt beziehungsweise in Seitenansicht dargestellt und man erkennt ein mit einer Ausnehmung 16 und zirkulären Nut 16' versehenes Lagerteil 14 mit daran angeordneter Wand 18. An der Wand 18 ist das mit einer in axialer Richtung orientierten Bohrung 17 versehene zylindrische Anschlussteil 30 und daran die Führungsbahn 35 angeordnet. Das Lagerteil 14 ist mit einer zirkulären Ausnehmung 14' für den im Profilquerschnitt halbkreisförmigen Ring 22 (Fig.4) sowie mit mindestens zwei, vorzugsweise mit mehreren in Umfangsrichtung verteilt angeordneten Schlitzen 19 versehen (Fig.6), welche jeweils zur Aufnahme des Hebels 20 (Fig.4) ausgebildet sind.

In Fig.7 ist als weitere Variante das manuell betätigbare Instrument 75 gemäss Fig.4 dargestellt und man erkennt ein Teilstück des Gehäuses 10 mit dem daran angeordneten Verbindungsglied 25 sowie die demontiert dargestellte Funktionseinheit 50. Abweichend von dem Instrument 75 gemäss Fig. 4 ist das am vorderen Ende des Gehäuses 10 angeordnete Verbindungsglied 25 mit einem einzigen an dem Lagerteil 14 des Kopfstücks 15 in dem Schlitz 19 gelagerten Hebel 20 versehen. Das Gehäuse 10 ist ebenfalls mit zwei oder mehreren in Umfangsrichtung verteilt angeordneten Schnappgliedern 8 an dem Kopfstück 15 befestigt. Bei dem in Fig.7 dargestellten Ausführungsbeispiel ist der Hebel 20 an einem im Kopfstück 15 angeordneten Bolzen 24 gelagert und mittels des Mitnehmers 21 zwischen den beiden in axialer Richtung im Abstand zueinander an der ersten Stange 12 angeordneten zirkulären Halterungen 11 und 11' angeordnet. Bei Betätigung des Hebels 20 gemäss Pfeilrichtung P wird die erste Stange 12 in Pfeilrichtung X verschoben und infolge davon, bei montierter Funktionseinheit 50, mit dem Schiebebolzen 60 (Fig.3) in Eingriff gebracht.

In Fig.8 und Fig.9 ist das Kopfstück 15 für das Verbindungsglied 25 gemäss Fig.7 im Schnitt beziehungsweise in Seitenansicht dargestellt. Das Kopfstück 15 ist im wesentlichen analog dem Kopfstück 15 gemäss Fig.5 und Fig.6 ausgebildet und umfasst das Lagerteil 14 mit der Wand 18 sowie das daran angeordnete zylindrische Anschlussteil 30 mit der Führungsbahn 35. Abweichend von dem Kopfstück 15 gemäss Fig.5,6 ist bei dem Ausführungsbeispiel das Kopfstück 15 gemäss Fig.8 und Figur 9 mit nur einem Schlitz 19 sowie mit einer quer dazu orientierten Bohrung 23 für den Bolzen 24 versehen. An dem Bolzen 24 ist der mit der ersten Stange 12 wirkverbundene Hebel 20 (Fig.7) schwenkbar gelagert.

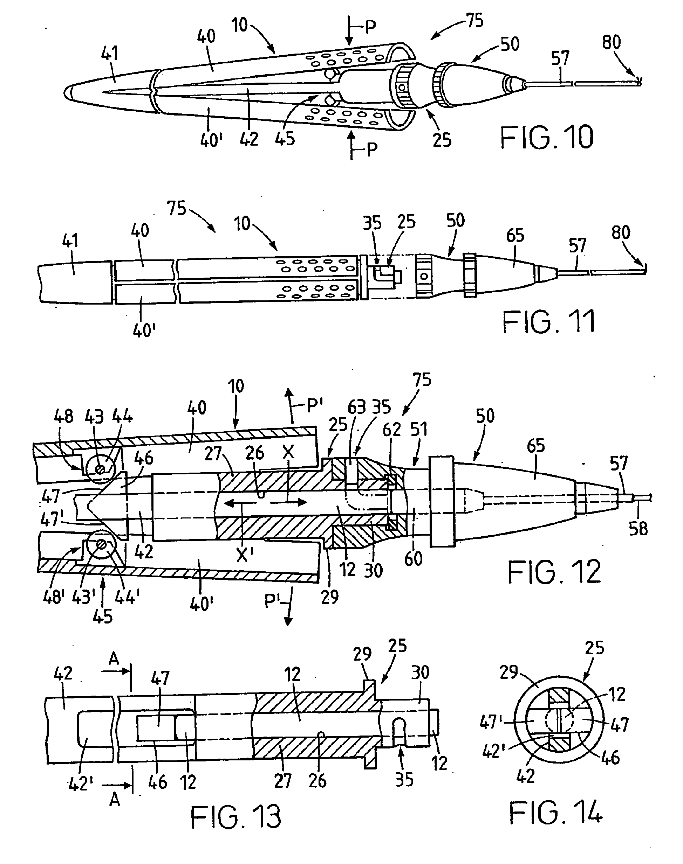

Fig.10 zeigt als drittes Ausführungsbeispiel das in räumlicher Ansicht und Fig.11 das in Ansicht dargestellte und manuell betätigbar ausgebildete Instrument 75. Das Instrument 75 umfasst das Gehäuse 10, das Verbindungsglied 25 sowie die Funktionseinheit 50 mit dem am distalen Ende angeordneten Werkzeug 80. Das Gehäuse 10 hat, wie in Fig. 10 dargestellt, zwei im Profilquerschnitt etwa halbkreisförmige Gehäuseteile 40 und 40' sowie eine an dem einen Ende daran angeordnete Verschlusskappe 41. Zwischen den beiden Gehäuseteilen 40,40' ist ein Tragarm 42 angeordnet, welcher im Bereich der Verschlusskappe 41 in nicht dargestellter Weise mit den beiden Gehäuseteilen 40 und 40' verbunden ist. Im vorderen Bereich ist zwischen den beiden Gehäuseteilen 40 und 40' eine Betätigungsvorrichtung 45 sowie das an dem Tragarm 42 befestigte Verbindungsglied 25 angeordnet. Bei demontierter Funktionseinheit 50 werden die beiden Gehäuseteile 40 und 40' infolge der eigenen federelastischen Rückstellkraft, wie in Fig.11 dargestellt, selbsttätig geschlossen.

Fig.12 zeigt das teilweise im Schnitt und in grösserem Massstab dargestellte vordere Teilstück des Instruments 75 gemäss Fig.10 und man erkennt das teilweise dargestellte Gehäuse 10 mit den beiden Gehäuseteilen 40 und 40' sowie die damit wirkverbundene Betätigungsvorrichtung 45. Weiterhin erkennt man das an dem Tragarm 42 angeordnete Verbindungsglied 25 mit der daran angeordneten Funktionseinheit 50. In dieser Stellung wird die erste Stange 12 abdichtend gegen den Dichtring 62 gedrückt. Die Betätigungsvorrichtung 45 umfasst je ein an der Innenseite der beiden Gehäuseteile 40 und 40' angeordnetes Lager 48 und 48'. Das einzelne Lager 48,48' ist zur Aufnahme einer an einer Achse 43 und 43' gelagerten Laufrolle 44 und 44' ausgebildet. Das Verbindungsglied 25 umfasst die in axialer Richtung von der Durchgangsbohrung 26 durchdrungene Führungshülse 27, den daran angeordneten Flansch 29 sowie das daran angeformte zylindrische Anschlussteil 30. In der Führungshülse 27 ist die erste Stange 12 angeordnet, welche im dargestellten Ausführungsbeispiel an dem einen Ende mit einem keilförmig ausgebildeten Gleitstück 46 versehen ist. Bei den manuell bewirkten und in Pfeilrichtung P (Fig.10) oder P' (Fig. 12) orientierten Bewegungen der beiden Gehäuseteile 40 und 40' gleiten die beiden Laufrollen 44 und 44' entlang der schrägen Gleitflächen 47 und 47' des Gleitstücks 46. Bei der in Pfeilrichtung P' (Fig.12) orientierten Bewegung der beiden Gehäuseteile 40 und 40' wird die erste Stange 12 in Pfeilrichtung X' und bei der in Pfeilrichtung P (Fig.10) orientierten Bewegung in Pfeilrichtung X bewegt.

Fig.13 zeigt ein in Draufsicht dargestelltes Teilstück des Tragarms 42 mit dem am vorderen Ende angeordneten Verbindungsglied 25 und man erkennt die Führungshülse 27 mit dem Flansch 29 sowie das daran angeformte Anschlussteil 30 mit der Führungsbahn 35. Die in der Durchgangsbohrung 26 angeordnete erste Stange 12 ist mit dem daran angeordneten Gleitstück 46 in einer in Längsrichtung des Tragarms 42 orientierten Ausnehmung 42' geführt. In Fig. 14 ist der Tragarm 42 gemäss der in Fig. 13 eingezeichneten Linie A-A im Schnitt dargestellt und man erkennt das an der Stange 12 angeordnete und in der Ausnehmung 42' zwangsläufig geführte Gleitstück 46 sowie den Flansch 29 des Verbindungsgliedes 25.

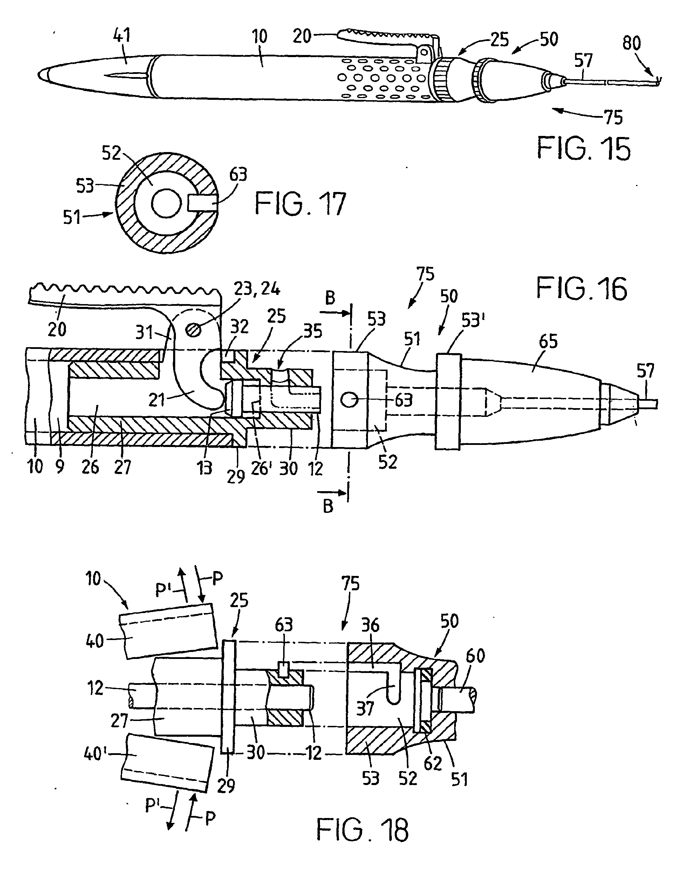

Fig.15 zeigt als viertes Ausführungsbeispiel ein in räumlicher Ansicht dargestelltes und manuell betätigbares Instrument 75. Das.Instrument 75 umfasst das mit der Verschlusskappe 41 versehene Gehäuse 10 sowie das daran angeordnete Verbindungsglied 25 für die Funktionseinheit 50 mit dem am distalen Ende angeordneten Werkzeug 80.

Fig.16 zeigt das in grösserem Massstab sowie teilweise im Schnitt dargestellte vordere Teilstück des Instruments 75 gemäss Fig.15 und man erkennt ein Teilstück des Gehäuses 10 sowie das daran angeordnete Verbindungsglied 25 mit der koaxial darin gelagerten ersten Stange 12. Die in Fig.16 demontiert und in Ansicht dargestellte Funktionseinheit 50 mit den einzelnen Elementen ist analog der vorstehend in Verbindung mit Fig.3 beschriebenen Funktionseinheit 50 ausgebildet. Das mit der in axialer Richtung orientierten Durchgangsbohrung 26.für die erste Stange 12 versehene Verbindungsglied 25 ist mit nicht dargestellten Mitteln, beispielsweise durch eine Kleb- oder Schweissverbindung, im Innenraum 9 des Gehäuses 10 befestigt. Das Verbindungsglied 25 umfasst die Führungshülse 27 mit dem Flansch 29 sowie das daran angeformte zylindrische Anschlussteil 30 mit der Führungsbahn 35.

Bei der in Fig.16 dargestellten Variante ist an dem Aussendurchmesser der Führungshülse 27 mindestens ein Lagerteil 31, vorzugsweise zwei jeweils mit einer Bohrung 23 versehene Lagerteile 31 in parallelem Abstand zueinander angeordnet. Zwischen den beiden Lagerteilen 31 ist der mit dem Mitnehmer 21 versehene Hebel 20 derart an dem Bolzen 24 gelagert, dass der Mitnehmer 21 mit einem an der ersten Stange 12 angeformten Kopfstück 13 wirkverbunden ist. Die in axialer Richtung orientierte und von dem Hebel 20 beziehungsweise Mitnehmer 21 bewirkte Bewegung der ersten Stange 12 ist durch einen in der Durchgangsbohrung 26 vorgesehenen Anschlag 26' begrenzt. Das Gehäuse 10 hat zum Einführen des mit einem oder zwei Lagerteilen 31 versehenen Verbindungsgliedes 25 eine schlitzförmig in axialer Richtung orientierte Ausnehmung 32.

Fig.17 zeigt das gemäss der in Fig. 16 eingezeichneten Linie B-B im Schnitt dargestellte Gehäuseteil 51 der Funktionseinheit 50 und man erkennt das kreisringförmige Teilstück 53 sowie den Rasterzapfen 63. Der Rasterzapfen 63 reicht mit dem einen Ende bis in den Innenraum 52 des Gehäuseteils 51 und ist mit dem anderen Ende in der Wand des Teilstücks 53 mit nicht dargestellten Mitteln befestigt.

In Fig.18 ist als fünftes Ausführungsbeispiel ein Teilstück des Instruments 75 dargestellt, bei welchem in der Ausnehmung 52 des Gehäuses 51 die mit einer in axialer Richtung orientierten ersten Nut 36 sowie mit einer in Umfangsrichtung orientierten zweiten Nut 37 versehene Führungsbahn 35 angeordnet ist. Der zum Einführen in die Führungsbahn 35 vorgesehene Rasterzapfen 63 ist bei diesem Ausführungsbeispiel an dem zylindrischen Anschlussteil 30 des mit dem Flansch 29 versehenen Verbindungsgliedes 25 angeordnet und mit nicht dargestellten Mitteln befestigt.

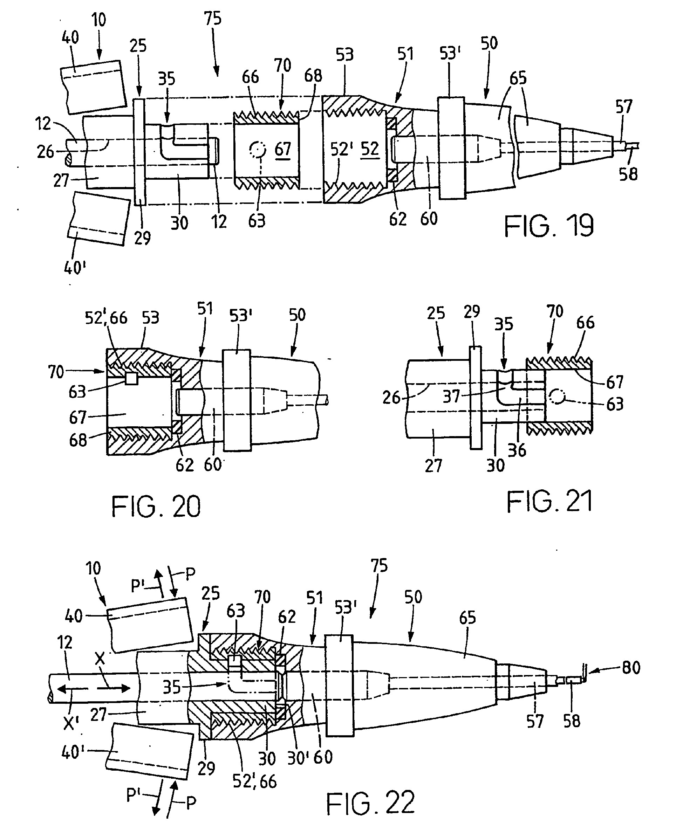

In Fig.19 ist als Sprengzeichnung ein weiteres Ausführungsbeispiel des Instruments 75 dargestellt und man erkennt das Gehäuse 10 mit dem Verbindungsglied 25 sowie ein mit einem Aussengewinde 66 versehenes Zwischenstück 70 zum lösbaren Verbinden der Funktionseinheit 50 an dem als Handgriff ausgebildeten Gehäuse 10. Das mit den beiden Gehäuseteilen 40 und 40' versehene Gehäuse 10 ist analog dem Gehäuse gemäss Fig.10 bis Fig.12 ausgebildet. Zwischen den beiden Gehäuseteilen 40 und 40' ist das mit der Führungshülse 27, dem Anlageflansch 29 sowie dem zylindrischen Anschlussteil 30 versehene Verbindungsglied 25 angeordnet. In der Führungshülse 27 beziehungsweise Bohrung 26 ist die mittels der in Fig.12 dargestellten Betätigungsvorrichtung 45 in axialer Richtung verschiebbare erste Stange 12 angeordnet. Weiterhin erkennt man die an dem zylindrischen Anschlussteil 30 des Verbindungsgliedes 25 angeordnete Führungsbahn 35, welche in Verbindung mit den Figuren 23 bis 25 näher beschrieben wird.

Die in Fig. 19 dargestellte Funktionseinheit 50 umfasst das teilweise im Schnitt dargestellte Gehäuse 51 mit der daran angeordneten Haube 65 sowie die in der Ausnehmung 52 angeordnete Dichtung 62. Abweichend von den vorstehend beschriebenen Funktionseinheiten 50 ist bei der in Fig. 19 dargestellten Funktionseinheit 50 die Ausnehmung 52 mit einem Innengewinde 52' versehen. Die mit dem Innengewinde 52' versehene Ausnehmung 52 ist zur schraubbaren Aufnahme des Zwischenstücks 70 ausgebildet. In dem Gehäuse 51 ist weiterhin der in axialer Richtung orientierte und mit der röhrchenförmigen Sonde 57 wirkverbundene Schiebebolzen 60 angeordnet. In der röhrchenförmigen Sonde 57 ist die Stange 58 angeordnet, welche am distalen Ende mit dem hier nicht dargestellten Werkzeug versehen ist. Zur Betätigung des Werkzeuges ist der Schiebebolzen 60 zusammen mit der röhrchenförmigen Sonde 57 relativ zu der zweiten Stange 58 in axialer Richtung verschiebbar.

Das in Fig.19 im Schnitt dargestellte und als zylindrischer Hülsenkörper ausgebildete Zwischenstück 70 ist mit dem Aussengewinde 66 sowie mit einer in axialer Richtung orientierten Durchgangsbohrung 67 versehen. In der Wand 68 des Zwischenstücks 70 ist der teilweise in die Durchgangsbohrung 67 ragender Zapfen 63 angeordnet und mit nicht dargestellten Mitteln befestigt.

Bei dem in Fig.20 dargestellten Anwendungsbeispiel ist das Zwischenstück 70 in das Innengewinde 52' der Funktionseinheit 50 eingeschraubt. Die beiden Teile 50 und 70 bilden hierbei eine Baueinheit, welche für die Montage an dem Gehäuse 10 auf den zylindrischen Anschlussteil 30 des Verbindungsgliedes 25 aufgeschoben wird. Bei der in axialer Richtung orientierten Bewegung der Funktionseinheit 50 wird der Zapfen 63 bis zu einem Anschlag 37' (Fig.24) in die Führungsbahn 35 geschoben und in der Endstellung zusammen mit dem eingeschraubten Zwischenglied 70 relativ zu dem Verbindungsglied 25 um die nicht näher dargestellte Längsachse gedreht bis der Zapfen 63 in eine Ausnehmung 39 (Fig.25) der Führungsbahn 35 formschlüssig einrastet.

Bei dem in Fig.21 dargestellten Anwendungsbeispiel wird das Zwischenstück 70 in axialer Richtung auf den zylindrischen Anschlussteil 30 des Verbindungsgliedes 25 geschoben bis der Zapfen 63 am Anschlag 37' der Führungsbahn 35 anliegt. In der Endstellung wird das Zwischenstück 70 relativ zu dem zylindrischen Anschlussteil 30 des Verbindungsgliedes 25 um die nicht dargestellte Längsachse gedreht bis der Zapfen 63 in die Ausnehmung 39 (Fig.25) der Führungsbahn 35 formschlüssig einrastet. In der Endstellung bildet das am ersten Gehäuse 10 angeordnete Verbindungsglied 25 mit dem Zwischenstück 70 eine Einheit. Bei diesem Anwendungsbeispiel wird die mit dem Innengewinde 52' versehene Funktionseinheit 50 auf das mit dem Aussengewinde 66 versehene Zwischenstück 70 aufgeschraubt.

Fig.22 zeigt das teilweise im Schnitt und vorstehend in Verbindung mit den Figuren 19 bis 21 beschriebene Instrument 75 in zusammengebautem Zustand und man erkennt das teilweise dargestellte Gehäuse 10 mit den beiden relativ zueinander bewegbaren Gehäuseteilen 40 und 40' sowie das Verbindungsglied 25 und das Zwischenstück 70 mit der aufgeschraubten Funktionseinheit 50. In dieser Stellung steht der Zapfen 63 mit der an dem zylindrischen Anschlussteil 30 des Verbindungsgliedes 25 vorgesehenen Führungsbahn 35 und die erste Stange 12 mit dem Schiebebolzen 60 der Funktionseinheit 50 für die Betätigung des Werkzeuges 80 in Wirkverbindung. In dieser Stellung wird die Stirnseite 30' des Anschlussteils 30 abdichtend gegen den Dichtring 62 der Funktionseinheit 50 gepresst.

Bei der um die Längsachse mit entsprechendem Bogenwinkel in Umfangsrichtung orientierten Drehbewegung des Gehäuses 10 relativ zu der Funktionseinheit 50 oder umgekehrt wird eine formschlüssige und stabile Verbindung der einzelnen Elemente und somit eine sichere Handhabung des vorstehend beschriebenen und in den einzelnen Figuren dargestellten Instruments 75 erreicht.

Infolge der manuell an den beiden Gehäuseteilen 40 und 40' gemäss Pfeilrichtung P wirkenden Kraft wird mittels der im Gehäuse 10 angeordneten Betätigungsvorrichtung 45 (Fig.12) die erste Stange 12 mit dem Schiebebolzen 60 gemäss Pfeilrichtung X verschoben und infolge davon das an der Funktionseinheit 50 angeordnete Werkzeug 80 für den ophthalmologischen Eingriff betätigt. Bei der in Pfeilrichtung P' orientierten Rückstellbewegung der beiden Gehäuseteile 40 und 40' wird der Schiebebolzen 60 mit der ersten Stange 12 in Pfeilrichtung X' bewegt.

Fig.23 zeigt ein in räumlicher Ansicht dargestelltes Teilstück des Verbindungsgliedes 25 und man erkennt den Flansch 29 mit dem angeformten und mit der Durchgangsbohrung 26 versehenen zylindrischen Anschlussteil 30 sowie die daran angeordnete Führungsbahn 35. Die Führungsbahn 35 hat ausgehend von der Stirnseite 30' des Anschlussteils 30 die in axialer Richtung orientierte erste Nut 36 sowie die etwa quer dazu teilweise in Umfangsrichtung orientierte zweite Nut 37. Die zweite Nut 37 ist durch eine etwa dem Zapfen 63 entsprechend halbkreisförmig ausgebildete Ausnehmung 39 begrenzt.

In Fig.24 ist als bevorzugtes Ausführungsbeispiel das Verbindungsglied 25 in Ansicht dargestellt und man erkennt den mit der Führungsbahn 35 versehenen zylindrischen Anschlussteil 30. Das Anschlussteil 30 hat ausgehend von dem Flansch 29 bis zu der Stirnseite 30' eine Länge C von 6 mm und einen äusseren Durchmesser D von 4,5 mm. Die in axialer Richtung das Verbindungsglied 25 durchdringende Bohrung 26 hat einen Durchmesser D' von 2,4 mm. Die ausgehend von der Stirnseite 30' mit einem Abstand A von 4,5 mm bis zu der Anschlagkante 37' reichende erste Nut 36 hat eine Breite B von 1,5 mm.

Fig.25 zeigt das in Form einer zeichnerischen Abwicklung (development of the surface) dargestellte Anschlussteil 30 mit der Führungsbahn 35 und man erkennt die ausgehend von der Stirnseite 30' in axialer Richtung orientierte erste Nut 36 sowie die etwa quer dazu orientierte zweite Nut 37. Die zweite Nut 37 ist durch die etwa halbkreisförmige und dem Durchmesser des Rasterzapfens 63 entsprechend ausgebildete Ausnehmung 39 begrenzt. Am Übergang von der ersten Nut 36 zu der zweiten Nut 37 ist ein in Richtung der zweiten Nut orientierter und konvex bogenförmig ausgebildeter erster Nocken 38 und am Ende der zweiten Nut 37 ein der Ausnehmung 39 zugeordneter konvex bogenförmig ausgebildeter zweiter Nocken 38' vorgesehen.

In Fig.25 ist das Einführen des Rasterzapfens 63 in die Führungsbahn 35 schematisch und in mehreren Phasen dargestellt. In einer ersten Phase gleitet der Rasterzapfen 63 entlang der ersten Nut 36 in Richtung der zweiten Nut 37. Sobald der Rasterzapfen 63 gegen die eine Innenwand 37' der zweiten Nut 37 stösst erfolgt die um die Längsachse orientierte Drehbewegung, infolge dessen der Rasterzapfen 63 über den ersten Nocken 38 bewegt und anschliessend in die zweite Führungsbahn 37 gelangt. Anschliessend wird der Rasterzapfen 63 entlang der einen Innenwand 37' oder entlang der gegenüberliegenden und schräg geneigt dazu ausgebildeten anderen Innenwand 37" bewegt. Sobald der Rasterzapfen 63 über den zweiten Nocken 38' bewegt wird rastet dieser mit exakt definierter Endstellung formschlüssig in die Ausnehmung 39 ein. Zwischen den beiden in Umfangsrichtung im Abstand zueinander angeordneten Nocken 38 und 38' der zweiten Führungsbahn 37 ist der Rasterzapfen 63 zur Vermeidung einer Klemmwirkung zwischen den beiden Innenwänden 37' und 37" mit geringem Spiel frei beweglich geführt.

Wie in Fig.25 dargestellt, ist die zweite Wand 37" ausgehend von dem ersten Nocken 38 in Richtung der Ausnehmung 39 in Bezug auf die erste Wand 37' geneigt ausgebildet. Zwischen den beiden in Umfangsrichtung des Anschlussteils 30 im Abstand zueinander angeordneten Nocken 38 und 38' der zweiten Führungsbahn 37 ist der Zapfen 63 zur Vermeidung einer Klemmwirkung zwischen den beiden im Abstand zueinander angeordneten Wänden 37' und 37" mit geringem Spiel frei beweglich geführt und zwischen den beiden Nocken 38 und 38' gegen nicht beabsichtigtes Lösen gesichert.

Durch eine in entgegengesetzter Richtung und relativ zueinander orientierten Drehbewegung des Gehäuses 10 in Bezug auf die Funktionseinheit 50 oder umgekehrt, kann die formschlüssige Verbindung rasch und ohne zusätzliche Hilfsmittel gelöst und anschliessend die Funktionseinheit 50 von dem zylindrischen Anschlussteil 30 des Verbindungsgliedes 25 in axialer Richtung abgezogen und entfernt werden.

Die vorstehend in Verbindung mit den Figuren 1 bis 25 im einzelnen beschriebenen Ausführungsbeispiele, Varianten und Anwendungsbeispiele sind nicht auf die einzelnen Funktionselemente beschränkt. Ohne den Grundgedanken der Erfindung zu verlassen sind weitere zweckmässige Ausgestaltungen und Anwendungsbeispiele ebenfalls möglich. Erfindungswesentlich ist, dass das vorzugsweise als Handgriff ausgebildete Gehäuse 10 durch eine in axialer Richtung orientierte Schiebebewegung und in der Endstellung durch eine um die Längsachse orientierte Drehbewegung um einen Winkel von etwa 90° ohne zusätzliche Hilfsmittel mit der Funktionseinheit 50 formschlüssig verbindbar ist. In der Endstellung ist infolge motorisch oder manuell betätigbarer Antriebsmittel die im Gehäuse 10 angeordnete erste Stange 12 mit dem in der Funktionseinheit 50 angeordneten Schiebebolzen 60 zur Betätigung des in den Figuren 1,2,10,11,15 und 22 schematisch dargestellten und am distalen Ende der Funktionseinheit 50 angeordneten Werkzeuges 80 (Schneid-, Greif- oder Klemmelement) wirkverbunden.

## Patentansprüche

1. Vorrichtung zum Verbinden einer lösbar an einem Gehäuse (10) angeordneten Funktionseinheit (50) mit einem daran angeordneten motorisch oder manuell betätigbaren Werkzeug (80) zur Durchführung ophthalmologischer Eingriffe, wobei ein an dem Gehäuse (10) angeordnetes Verbindungsglied (25) mit daran angeordnetem Anschlussteil (30) zur Aufnahme der Funktionseinheit (50), welche in einer an dem Anschluss-teil (30) oder in der Funktionseinheit (50) angeordneten nut- oder schlitzförmigen Führungsbahn (35) bewegbar und in axialer Endstellung durch eine relativ zu dem Gehäuse (10) um die gemeinsame Längsachse orientierte Drehbewegung formschlüssig an dem Anschlussteil (30) gehalten ist, derart, dass eine in dem Verbindungsglied (25) gelagerte sowie mit mindestens einem Antrieb zusammenwirkende Stange (12) in axialer Richtung verschiebbar ist und in der Funktionseinheit (50) ein Schiebebolzen (60) zur Betätigung des Werkzeuges (80) angeordnet ist, **dadurch gekennzeichnet, dass** die Stange (12) mit dem Schiebebolzen (60) durch eine am Schiebebolzen (60) angeordnete Druckfeder (56) wirkverbunden ist.

2. Vorrichtung nach Anspruch 1, **dadurch gekennzeichnet, dass** die an dem Anschlussteil (30) des Verbindungsgliedes (25) angeordnete nut- oder schlitzförmige Führungsbahn (35) eine in axialer Richtung orientierte erste Nut (36) sowie eine mindestens teilweise in Umfangsrichtung orientierte und durch eine halbkreisförmige Ausnehmung (39) begrenzte zweite Nut (37) aufweist, und dass die Funktionseinheit (50) mit einem daran angeordneten Rasterzapfen (63) versehen ist, welcher in der Endstellung der Drehbewegung formschlüssig einrastend in der halbkreisförmigen Ausnehmung (39) angeordnet ist.

3. Vorrichtung nach Anspruch 2, **dadurch gekennzeichnet, dass** die an dem Anschlussteil (30) in Umfangsrichtung orientierte zweite Nut (37) zwei im Abstand zueinander in Umfangsrichtung orientierte Innenwände (37';37") aufweist, von welchen die eine der Stirnseite (30') zugewandte erste Innenwand (37") zwei im Abstand zueinander angeordnete und jeweils in Richtung der gegenüberliegenden zweiten Wand (37') konvex bogenförmig ausgebildete Rastnocken (38,38') aufweist.

4. Vorrichtung nach Anspruch 3, **dadurch gekennzeichnet, dass** die mit den beiden in Umfangsrichtung im Abstand zueinander angeordneten Rastnocken (38,38') versehene erste Innenwand (37") in Bezug auf die gegenüberliegende zweite Innenwand (37') in Richtung der halbkreisförmigen Ausnehmung (39) geneigt ausgebildet ist, so dass die zweite Nut (37) ausgehend von der ersten Nut (36) in Richtung der Ausnehmung (39) etwa konisch verjüngend ausgebildet ist.

5. Vorrichtung nach Anspruch 3, **dadurch gekennzeichnet, dass** der infolge der Drehbewegung von der ersten Nut (36) über den ersten Rastnocken (38) in die zweite Nut (37) geführte Rasterzapfen (63) zwischen den beiden gegenüberliegenden Innenwänden (37',37") frei beweglich in Richtung der Ausnehmung (39) geführt und darin durch den zweiten Rastnocken (38') gehalten ist.

6. Vorrichtung nach Anspruch 2, **dadurch gekennzeichnet, dass** das am Verbindungsglied (25) angeordnete Anschlussteil (30) zylindrisch ausgebildet und ausgehend von der Stirnseite (30') eine Länge (C) von 6 mm und einen äusseren Durchmesser (D) von 4,5 mm aufweist, und dass die ausgehend von der Stirnseite (30') in axialer Richtung orientierte erste Nut **(36)** eine Länge (A) von 4,5 mm und die zweite Nut (37) in Bezug auf die Nut (36) einen in Umfangsrichtung orientierten Bogenwinkel von etwa 75° bis 120°, vorzugsweise einen Bogenwinkel von 90° aufweist.

7. Vorrichtung nach Anspruch 1, **dadurch gekennzeichnet, dass** die nut- oder schlitzförmige Führungsbahn (35) in einer Ausnehmung (52) der Funktionseinheit (50) angeordnet und diese in axialer Richtung auf das Anschlussteil (30) des Verbindunggliedes (25) schiebbar sowie in axialer Endstellung durch eine relativ zu dem Gehäuse (10) um die gemeinsame Längsachse orientierte Drehbewegung formschlüssig an dem Anschlussteil (30) gehalten ist und gleichzeitig die Stange (12) mit dem zur Betätigung des Werkzeuges (80) in der Funktionseinheit (50) angeordneten Schiebebolzen (60) wirkverbunden ist.

8. Vorrichtung nach Anspruch 1, **gekennzeichnet durch** ein zur schraubbaren Aufnahme der Funktionseinheit (50) ausgebildetes Zwischenglied (70), welches mit einem daran angeordneten Rasterzapfen (63) in der Führungsbahn (35) des Anschlussteils (30) in axialer Richtung bewegbar und in axialer Endstellung **durch** eine relativ zu dem am Gehäuse (10) angeordneten Verbindungsglied (25) um die gemeinsame Längsachse orientierte Drehbewegung formschlüssig an dem Anschlussteil (30) gehalten ist.

9. Vorrichtung nach Anspruch 1, **dadurch gekennzeichnet, dass** die koaxial in dem Verbindungsglied (25) angeordnete Stange (12) von einem in dem Gehäuse (10) angeordneten Antrieb (6) für die Betätigung des an der Funktionseinheit (50) angeordneten Werkzeuges (80) in axialer Richtung verschiebbar ist, und dass der mit einer Energiequelle (1) wirkverbundene Antrieb (6) elektromotorisch, hydraulisch oder pneumatisch betätigbar ist.

10. Vorrichtung nach Anspruch 1, **dadurch gekennzeichnet, dass** die koaxial in dem Verbindungsglied (25) angeordnete Stange (12) mittels einer in dem Gehäuse (10) angeordneten Batterie (6') für die Betätigung des an der Funktionseinheit (50) angeordneten Werkzeuges (80) in axialer Richtung verschiebbar ist.

11. Vorrichtung nach Anspruch 1, **dadurch gekennzeichnet, dass** das an dem Gehäuse (10) angeordnete Verbindungsglied (25) ein Kopfstück (15) mit daran angeordnetem Anschlussteil (30) umfasst, wobei an dem Kopfstück (15) mindestens ein manuell betätigbarer Hebel (20) gelagert ist, welcher mit einem daran angeordneten Mitnehmer (21) mit der in axialer Richtung verschiebbaren Stange (12) für die Betätigung des an der Funktionseinheit (50) angeordneten Werkzeuges (80) wirkverbunden ist.

12. Vorrichtung nach Anspruch 11, **gekennzeichnet durch** mehrere in Umfangsrichtung verteilt an dem Kopfstück (15) des Verbindungsgliedes (25) angeordnete und jeweils in einer schlitzförmigen Ausnehmung (19) gelagerte Hebel (20), wobei jeder einzelne Hebel (20) mit dem daran angeordneten Mitnehmer (21) zwischen zwei im Abstand zueinander angeordnete Halteteile (11,11') mit der Stange (12) wirkverbunden sind.

13. Vorrichtung nach Anspruch 1, **dadurch gekennzeichnet, dass** das Gehäuse (10) zwei an dem einen Ende miteinander verbundene und an dem anderen Ende relativ zueinander bewegbare längliche Gehäuseteile (40,40'), einen dazwischen angeordneten Tragarm (42) sowie das am distalen Ende daran befestigte Verbindungsglied (25) umfasst, und dass die koaxial darin gelagerte und mit einer Antriebsmechanik (45) wirkverbundene Stange (12) beim manuellen Zusammendrücken der beiden Gehäuseteile (40,40') in axialer Richtung verschiebbar und dabei mit dem Schiebebolzen (60) und Werkzeug (80) der formschlüssig am Anschluss-teil (30) angeordneten Funktionseinheit (50) in Eingriff bringbar ist.

14. Vorrichtung nach Anspruch 13, **dadurch gekennzeichnet, dass** die in dem Verbindungsglied (25) gelagerte Stange (12) ein keilförmiges Gleitstück (46) aufweist, welches mit an den Gehäuseteilen (40, 40') gelagerten Rollen (44,44') derart zusammenwirkt, dass beim manuellen Zusammendrücken der beiden Gehäuseteile (40,40') die Stange (12) in axialer Richtung verschiebbar und dabei mit dem Werkzeug (80) der formschlüssig am Anschlussteil (30) angeordneten Funktionseinheit (50) in Eingriff bringbar ist.

15. Vorrichtung nach Anspruch 14, **dadurch gekennzeichnet, dass** der zwischen den beiden Gehäuseteilen (40,40') angeordnete Tragarm (42) eine in Längsrichtung orientierte Ausnehmung (42') aufweist, in welcher das an der Stange (12) angeordnete keilförmige Gleitstück (46) in axialer Richtung geführt und gegen Verdrehung gesichert ist.

16. Vorrichtung nach Anspruch 1, **dadurch gekennzeichnet, dass** Verbindungsglied (25) eine in dem Gehäuse (10) angeordnete Führungshülse (27), einen daran gelagerten Hebel (20) sowie das Anschlussteil (30) zur formschlüssigen Verbindung der Funktionseinheit (50) umfasst, und dass der Hebel (20) mit daran angeordnetem Mitnehmer (21) mit der in axialer Richtung verschiebbaren Stange (12) für die Betätigung des an der Funktionseinheit (50) angeordneten Werkzeuges (80) wirkverbunden ist.

17. Vorrichtung nach Anspruch 16, **dadurch gekennzeichnet, dass** die in axialer Richtung orientierte Bewegung der in der Führungshülse (27) des Verbindungsgliedes (25) angeordneten Stange (12) durch ein daran angeordnetes und mit dem Mitnehmer (21) des Hebel (20) zusammenwirkendes Kopfstück (13) sowie durch einen in der Führungshülse (27) vorgesehenen Anschlag (26') begrenzt und der Durchgangsbohrung (26) gehalten ist.

18. Vorrichtung nach Anspruch 1, **gekennzeichnet durch** die Verwendung des zur Aufnahme eines elektromotorisch, hydraulisch oder pneumatisch betätigbaren Antriebs (6) ausgebildeten Gehäuses (10) mit dem daran angeordneten Verbindungsglied (25) als erste Baueinheit und der mit dem Werkzeug (80) versehenen Funktionseinheit (50) als zweite Baueinheit, wobei die beiden Baueinheiten **durch** axiales Zusammenfügen sowie um die gemeinsame Längsachse relativ zueinander orientierte Drehbewegung formschlüssig miteinander und die Stange (12) und der Schiebebolzen (60) zur Betätigung des Werzeuges (80) wirkverbunden sind.

19. Vorrichtung nach Anspruch 1, **gekennzeichnet durch** die Verwendung des zur Aufnahme eines von einer Batterie (6') betätigbaren Antriebs (6) ausgebildeten Gehäuses (10) mit dem daran angeordneten Verbindungsglied (25) als erste Baueinheit und der mit dem Werkzeug (80) versehenen Funktionseinheit (50) als zweite Baueinheit, wobei die beiden Baueinheiten durch axiales Zusammenfügen sowie um die gemeinsame Längsachse relativ zueinander orientierte Drehbewegung formschlüssig miteinander und die Stange (12) und der Schiebebolzen (60) zur Betätigung des Werzeuges (80) wirkverbunden sind.

20. Vorrichtung nach Anspruch 1, **gekennzeichnet durch** die Verwendung des mit mindestens einem manuell betätigbaren Hebel (20) versehenen Gehäuse (10) mit dem Verbindungsglied (25) als erste Baueinheit und der mit dem Werkzeug (80) versehenen Funktionseinheit (50) als zweite Baueinheit, wobei die beiden Baueinheiten **durch** axiales Zusammenfügen sowie um die gemeinsame Längsachse relativ zueinander orientierte Drehbewegung formschlüssig und die Stange (12) sowie der Schiebebolzen (60) zur Betätigung des Werzeuges (80) miteinander wirkverbunden sind.

21. Vorrichtung nach Anspruch 1, **gekennzeichnet durch** die Verwendung des mit zwei relativ zueinander zusammendrückbaren Gehäuseteilen (40;40') versehenen Gehäuses (10) mit dem daran angeordneten Verbindungsglied (25) als erste Baueinheit und der mit dem Werkzeug (80) versehenen Funktionseinheit (50) als zweite Baueinheit, wobei die beiden Baueinheiten **durch** axiales Zusammenfügen sowie um die gemeinsame Längsachse relativ zueinander orientierte Drehbewegung formschlüssig und die Stange (12) sowie der Schiebebolzen (60) zur Betätigung des Werzeuges (80) miteinander wirkverbunden sind.

22. Vorrichtung nach Anspruch 1, **gekennzeichnet durch** die Verwendung des Gehäuses (10) mit dem daran angeordneten Verbindungsglied (25) als erste Baueinheit und der mit einem eingeschraubten Zwischenglied (70) sowie mit dem Werkzeug (80) versehenen Funktionseinheit (50) als zweite Baueinheit, wobei die beiden Baueinheiten **durch** eine axiales Zusammenfügen sowie um die gemeinsame Längsachse relativ zueinander orientierte Drehbewegung formschlüssig und die Stange (12) spwoe der Schiebebolzen (60) zur Betätigung des Werzeuges (80) miteinander wirkverbunden sind.

23. Vorrichtung nach Anspruch 1, **gekennzeichnet durch** die Verwendung des Gehäuses (10) mit dem Verbindungsglied (25) und einem formschlüssig daran angeordneten und mit einem Aussengewinde (66) versehenen Zwischenglied (70) als erste Baueinheit und der mit einem Innengewinde (52') sowie mit dem Werkzeug (80) versehenen Funktionseinheit (50) als zweite Baueinheit, wobei die beiden Baueinheiten **durch** die Schraubverbindung miteinander und die Stange (12) sowie der Schiebebolzen (60) zur Betätigung des Werzeuges (80) miteinander wirkverbunden sind.

24. Vorrichtung nach Anspruch 1, **gekennzeichnet durch** die Verwendung der mit einem Innengewinde (52') versehenen Funktionseinheit (50) als erste Baueinheit und des Gehäuses (10) mit dem Verbindungsglied (25) sowie dem formschlüssig daran gehaltenen und mit dem Aussengewinde (66) versehenen Zwischenglied (70) als zweite Baueinheit, wobei die beiden Baueinheiten **durch** die Schraubverbindung miteinander und die Stange (12) sowie der Schiebebolzen (60) zur Betätigung des Werzeuges (80) miteinander wirkverbunden sind.

## Claims

1. Device for removable connection of a functional unit (50) provided with a manually or motorically operable surgical tool (80) to a housing (10) for performing ophthalmologic operations, comprising a connecting member (25) disposed in the housing (10), an adapter (30) disposed at the connecting member (25) configured for engagement with the functional unit (50, which unit is movable in a slot-shaped guide track (35) provided in one of the adapter (30) or the functional unit (50) and which unit is engaged in an axial end position in a form-fitting connection at the adapter (30) by a rotation oriented relatively to the housing (10) along the mutual axis such that a rod (12) mounted in the adapter (30) and co-operating at least with a drive is movable in axial direction, and a sliding pin (60) is disposed in the functional unit (50) for actuating the surgical tool (80), **characterized in that** the rod (12) is operatively engaged with the sliding pin (60) by means of a pressure spring (56) disposed at the sliding pin (60).

2. Device according to claim 1, **characterized in that** the slot-shaped guide track (35) disposed at the adapter (30) of the connecting member (25) is configured with a first groove (36) extending in axial direction and a second groove (37) extending substantially transverse to the first groove and bordered by a semi-circular recess (39), and wherein the functional unit (50) is provided with a locking peg (63) for traveling in the slot-shaped guide track such that after a rotational motion of the functional unit relative to the housing (10) the locking peg is locked at the semicircular recess (39) whereby the functional unit is form-fittingly connected to the housing.

3. Device according to claim 2, **characterized in that** the second groove (37) includes first and second inner walls (37',37") extending circumferentially and at a distance from each other and wherein the first inner wall (37") facing a front side (30') is provided with two curved and arc-shaped locking cams (38,38') oriented at a distance from each other with a curved part facing in the direction of the second inner wall (37').

4. Device according to claim 3, **characterized in that** the first inner wall (37") relative to the second wall (37') facing the first wall is sloped in the direction of the semi-circular recess (39), so that the second groove (37) starting from the first groove (36) is tapered in direction of the recess.

5. Device according to claim 3, **characterized in that** the first and second grooves (36,37) are dimensioned for clearance of the locking peg (63) when the locking peg is guided on the inner walls (37',37") in the direction of the recess (39) and thus held in the recess by the second locking cam (38').

6. Device according to claim 2, **characterized in that** the adapter (30) disposed at the connecting member (25) is cylindrical-shaped and starting from the front side (30') has a length dimension of 6 mm and an outer diameter of 4,5 mm, and the first groove (36) starting from the front side (30') and oriented in axial direction has a length of 4,5 mm and the second groove (37) is positioned at an arced angle in the range from 75° to 120°, preferably in the range of 90° relative to the first groove.

7. Device according to claim 1, **characterized in that** the slot-shaped guide track (35) is disposed in a recess (52) of the functional unit (50) and axially movable relative to the housing (10) onto the adapter (30) of the connecting member (25) into an axial end position for a form-fitting engagement at the adapter, such that upon engagement, the rod (12) can be at the same time operatively engaged with the sliding pin (60) disposed in the functional unit for actuation of the surgical tool (80).

8. Device according to claim 1, **characterized in that** an intermediary member (70) for threadedly receiving the functional unit (50) and a locking peg (63), and disposed at the intermediary member for insertion into and locking engagement in the slot-shaped guide track (35) in the adapter (30) when the intermediary member is axially moved into an end position relative to the connecting member (25) of the housing (10) for a from-fitting connection to the adapter.

9. Device according to claim 1, **characterized in that** the drive (6) disposed in the housing (10) is powered electrically, hydraulic or pneumatic by a power source (1) for providing an axial movement of the rod (12) to thereby actuate the surgical tool (80) mounted to the functional unit (50).

10. Device according to claim 1, **characterized in that** the drive disposed in the housing (10) is powered by a battery (6') for actuating the surgical tool (80) in axial movement.

11. Device according to claim 1, **characterized in that** the connecting member (25) includes a head piece (15) connected to the adapter (30) and at least one manually operable lever (20) supported at the head piece and including a catch (21) by which the head piece is brought into operative engagement with the axially movable the rod (12).

12. Device according to claim 11, **characterized in that** the head piece (15) is configured with slot-shaped recesses (19) in circumferential arrangement, each of the recesses configured for supporting a lever (20) when two or more levers are provided, and wherein each of the levers disposed with a catch (21) held between two retaining pieces (11,11') apart from each other at a distance is in operative engagement with the rod (12).

13. Device according to claim 1, **characterized in that** the housing (10) split longitudinally into two elongate housing parts (40,40') connected to each other at one end and movable relative to each other at another end includes a carrying arm (42) disposed there between with the connecting member (25) disposed at a distal end and with the rod (12) co-axially supported in the housing and connected to a drive mechanism (45), so that when the two housing parts are manually pressed together, the rod which is movable in axial direction, can be brought in operative engagement with the surgical tool (80) which is form-fittingly connected to the functional unit (50) disposed at the adapter (30).

14. Device according to claim 13, **characterized in that** the rod (12) supported in the connecting member (25) is provided with a wedge-shaped sliding member (46) for engagement with rollers (44,44') supported in the housing (10), and wherein the two housing parts (40,40') when pressed together can cause the rod (12) to slide in axial direction to thereby bring the rod into engagement with the sliding pin (60) and the surgical tool (80) which is form-fittingly connected to the functional unit (50) disposed at the adapter (30).

15. Device according to claim 14, **characterized in that** the carrying arm (42) is provided with a longitudinally oriented recess (42') in which the wedge-shaped sliding member (46) can be guided in axial direction for fixed rotative engagement.

16. Device according to claim 1, **characterized in that** the connecting member (25) includes a guide sleeve (27) disposed in the housing (10), a lever (20) supported at the sleeve (27) and the adapter (30) for a form-fitting connection with the functional unit (50), and wherein the lever is provided with a catch (21), which is in operative engagement with the axially sliceable rod (12) for engagement with the sliding pin (60) in operative engagement with the surgical tool (80) which is form-fittingly connected to the functional unit disposed at the adapter.

17. Device according to claim 16, **characterized in that** the guide sleeve (27) is configured with an axial bore (26) ending in a stop (26'), and wherein the rod (12) includes a head piece (13) for engagement with the catch (21) of the lever (20) such that upon movement of the lever the head piece (13) can bear upon the stop.

18. Device according to claim 1, **characterized in that** the housing (10), which is configured for receiving a drive mechanism (6) of the type selected from the group of electric motor drive, hydraulic drive and pneumatic drive is conceived as a first subassembly and the functional unit (50) which is provided with the surgical tool (80) is conceived as a second subassembly, and wherein the two subassemblies are brought into operative engagement by a rotational motion relative to each other about the longitudinal axis for providing a form-fitting connection with the rod (12) and the sliding pin (60), and with each other.

19. Device according to claim 1, **characterized in that** the housing (10) is configured for receiving a battery (6') for actuating a drive mechanism (6) and is conceived as a first subassembly and the functional unit (50) which is provided with the tool (80) is conceived as a second subassembly, and wherein the two subassemblies are brought into operative engagement by a rotational motion relative to each other about the longitudinal axis for providing a form-fitting connection with the rod (12) and the sliding pin (60), and with each other.

20. Device according to claim 1, **characterized in that** at least one a lever (20) is provided at the housing (10) for operative engagement with the axially movable rod (12), and wherein the housing is configured as a first subassembly and the functional unit (50) which is provided with the surgical tool (80) is conceived as a second subassembly, and wherein the two subassemblies are brought into operative engagement by a rotational motion relative to each other about the longitudinal axis for providing a form-fitting connection with the movable rod and the sliding pin (60), and with each other.

21. Device according to claim 1, **characterized in that** the housing (10) is split longitudinally into two elongate housing parts (40',40") and the connecting member (25) provided at the housing is conceived as a first subassembly and the functional unit (50) which is provided with the surgical tool (80) is conceived as a second subassembly, and wherein the two subassemblies are brought into operative engagement by a rotational motion relative to each other about the longitudinal axis for providing a form-fitting connection with the rod (12) and the sliding pin (60), and with each other.

22. Device according to claim 1, **characterized in that** the housing (10) including the connecting member (25) is conceived as a first subassembly and the functional unit (50) which is provided with the surgical tool (80) and is threadedly connected to an intermediary member (70) is conceived as a second subassembly, and wherein the two subassemblies are brought into operative engagement by a rotational motion relative to each other about the longitudinal axis for providing a form-fitting connection with the rod (12) and the sliding pin (60), and with each other.

23. Device according to claim 1, **characterized in that** the housing (10) including the connecting member (25) and an intermediary member (70) with an outer thread (66) for form-fitting engagement with the connecting member is conceived as a first subassembly and the functional unit (50) which is provided with an interior thread (52) threadedly connected to the intermediary member and provided with the surgical tool (80) is conceived as a second subassembly, and wherein the two subassemblies are brought into operative engagement by a rotational motion relative to each other about the longitudinal axis for providing a form-fitting connection with the rod (12) and the sliding pin (60), and with each other.

24. Device according to claim 1, **characterized in that** the functional unit (50) is provided with an inner thread (52') is conceived as a first subassembly and the housing (10) including the connecting member (25) and an intermediary member (70) provided with an outer thread (66) for providing a form-fitting threaded connection with the connecting member is conceived as second subassembly and wherein the two subassemblies are brought into operative engagement by a rotational motion relative to each other about the longitudinal axis for providing a form-fitting connection with the rod (12) and the sliding pin (60), and with each other.

## Revendications

1. Dispositif pour l'assemblage d'une unité fonctionnelle (50) disposée de manière amovible sur un boîtier (10) avec un outil (80) disposé dessus, pouvant être actionné par un moteur ou manuellement pour l'exécution d'opérations ophtalmologiques, dans lequel un élément d'assemblage (25) disposé sur le boîtier (10) est mobile avec une partie de raccordement (30) disposée dessus pour recevoir l'unité fonctionnelle (50), qui est mobile dans une piste de guidage (35) en forme de gorge ou de fente ménagée dans la partie de raccordement (30) ou l'unité de fonctionnelle (50) et retenue dans la position de fin de course axiale par un mouvement de rotation autour de l'axe longitudinal commun par rapport au boîtier (10) sur la partie de raccordement (30), de telle sorte qu'une tige (12) supportée dans l'élément d'assemblage (25) et coopérant avec au moins un entraînement puisse coulisser dans le sens axial et dans le quel un goujon coulissant (60) destiné à actionner l'outil (80) est disposé dans l'unité fonctionnelle (50), **caractérisé en ce que** la tige (12) est en liaison active avec le goujon coulissant (60) à l'aide d'un ressort de compression (56) disposé sur le goujon coulissant (60).

2. Dispositif selon la revendication 1, **caractérisé en ce que** la piste de guidage (35) en forme de gorge ou de fente disposée sur la partie de raccordement (30) de l'élément d'assemblage (25) possède une première gorge (36) orientée dans le sens axial et une deuxième gorge (37) orientée au moins partiellement dans le sens de la circonférence et délimitée par un creux en forme de demi-cercle (39), et **en ce que** l'unité fonctionnelle (50) est dotée d'un goujon d'engagement (63) disposé dessus, qui est disposé, dans la position finale du mouvement de rotation, en engagement positif dans le creux en forme de demi-cercle (39).

3. Dispositif selon la revendication 2, **caractérisé en ce que** la deuxième gorge (37)orientée dans le sens de la circonférence sur la partie de raccordement (30) possède deux parois intérieures (37', 37") écartées l'une de l'autre et orientées dans le sens de la circonférence, dont une première paroi intérieure (37") tournée vers la face d'extrémité (30') porte deux cames d'engagement (38, 38') disposées à distance l'une de l'autre et convexes en forme d'arc de cercle vers la deuxième paroi (37') opposée.

4. Dispositif selon la revendication 3, **caractérisé en ce que** la première paroi intérieure (37") portant les deux cames d'engagement (38, 38') disposées à distance l'une de l'autre est inclinée par rapport à la deuxième paroi intérieure (37') opposée en direction du creux en forme de demi-cercle (39), de sorte que la deuxième gorge (37) se resserre à peu près en forme de cône à partir de la première gorge (36) vers le creux (39).

5. Dispositif selon la revendication 3, **caractérisé en ce que** le goujon d'engagement (63) guidé, à la suite du mouvement de rotation, de la première gorge (36) par-dessus la première came d'engagement (38) dans la deuxième gorge (37) est guidé de manière librement mobile en direction du creux (39) entre les deux parois intérieures (37', 37") opposées et y est retenu par la deuxième came d'engagement (38').

6. Dispositif selon la revendication 2, **caractérisé en ce que** la partie de raccordement (30) disposée sur l'élément d'assemblage (25) est de forme cylindrique et présente, à partir de la face d'extrémité (30'), une longueur (C) de 6 mm et un diamètre extérieur (D) de 4,5 mm, et **en ce que** la première gorge (36) orientée dans le sens axial à partir de la face d'extrémité (30') a une longueur (A) de 4,5 mm et la deuxième gorge (37) forme par rapport à la gorge (36) un angle d'arc orienté dans le sens de la circonférence d'environ 75° à 120°, de préférence un angle d'arc de 90°.

7. Dispositif selon la revendication 1, **caractérisé en ce que** la piste de guidage (35) en forme de gorge ou de fente est disposée dans un creux (52) de l'unité fonctionnelle (50) et celle-ci peut coulisser sur la partie de raccordement (30) de l'élément d'assemblage (25) et est retenue dans la position finale axiale sur la partie de raccordement (30) en engagement positif par un mouvement de rotation par rapport au boîtier (10) autour de l'axe longitudinal commun et la tige (12) est en même temps en liaison active avec le goujon coulissant (60) disposé dans l'unité fonctionnelle (50) pour actionner l'outil (80).

8. Dispositif selon la revendication 1, **caractérisé en ce qu'**il comporte une pièce intercalaire (70) conçue pour recevoir par vissage l'unité fonctionnelle (50), qui est retenu de façon mobile dans le sens axial avec un goujon d'engagement (63) disposé dessus dans la piste de guidage (35) de la partie de raccordement (30) et en engagement positif sur la partie de raccordement (30), dans la position finale axiale, par un mouvement de rotation par rapport à l'élément d'assemblage (25) disposé sur le boîtier (10) ou inversement autour de l'axe longitudinal commun.

9. Dispositif selon la revendication 1, **caractérisé en ce que** la tige (12) coaxiale dans l'élément d'assemblage (25) peut être déplacée dans le sens axial par un entraînement (6) disposé dans le boîtier (10) pour actionner l'outil (80) disposé sur l'unité fonctionnelle (50), et **en ce que** l'entraînement (6) en liaison active avec une source d'énergie (1) est à actionnement électromoteur, hydraulique ou pneumatique.

10. Dispositif selon la revendication 1, **caractérisé en ce que** la tige (12) coaxiale dans l'élément d'assemblage (25) peut être déplacée dans le sens axial au moyen d'une batterie (6') disposée dans le boîtier (10) pour actionner l'outil (80) disposé sur l'unité fonctionnelle (50).

11. Dispositif selon la revendication 1, **caractérisé en ce que** l'élément d'assemblage (25) disposé sur le boîtier (10) comprend une partie de tête (15) qui porte la partie de raccordement (30), la partie de tête (15) supportant au moins un levier (20) à actionnement manuel qui est en liaison active avec un entraîneur (21) disposé dessus avec une tige (12) mobile dans le sens axial pour l'actionnement de l'outil (80) disposé sur l'unité fonctionnelle (50).

12. Dispositif selon la revendication 11, **caractérisé en ce qu'**il comporte plusieurs leviers (20) répartis dans le sens de la circonférence sur la partie de tête (15) de l'élément d'assemblage (25) et supportés chacun dans un creux (19) en forme de fente, chaque levier (20) étant en liaison active avec l'entraîneur (21) disposé dessus avec la tige (12) entre deux parties de maintien (11, 11') disposées à distance l'une de l'autre.

13. Dispositif selon la revendication 1, **caractérisé en ce que** le boîtier (10) possède deux parties de boîtier (40,40') allongées, reliées entre elles à une extrémité et mobiles l'une par rapport à l'autre à l'autre extrémité, un bras de support (42) disposées entre celles-ci et l'élément d'assemblage (25) fixé dessus à l'extrémité distale, et **en ce que** la tige (12) supportée de façon coaxiale à l'intérieur et en liaison active avec un mécanisme d'entraînement (45) peut être déplacée dans le sens axial lors du rapprochement manuel des deux parties de boîtier (40,40') et peut ainsi être mise en prise avec le goujon coulissant (60) et l'outil (80) de l'unité fonctionnelle (50) disposée en engagement positif sur l'élément de raccordement (30).

14. Dispositif selon la revendication 13, **caractérisé en ce que** la tige (12) supportée dans l'élément d'assemblage (25) possède une coulisse (46) en forme de coin, qui coopère avec des galets (44, 44') disposés sur les parties de boîtier (40, 40') de telle sorte que lors du rapprochement manuel des deux parties de boîtier (40, 40'), la tige (12) puisse être déplacée dans le sens axial et ainsi mise en prise avec l'outil (80) de l'unité fonctionnelle (50) disposée en engagement positif sur la partie de raccordement (30).

15. Dispositif selon la revendication 14, **caractérisé en ce que** le bras de support (42) disposé entre les deux parties de boîtier (40, 40') possède un creux (42') orienté dans le sens longitudinal, dans lequel la coulisse (46) en forme de coin disposée sur la tige (12) est guidée dans le sens axial et fixée de façon à empêcher sa rotation.

16. Dispositif selon la revendication 1, **caractérisé en ce que** l'élément d'assemblage (25) comprend une douille de guidage (27) disposée dans le boîtier (10), un levier (20) supporté dessus et la partie de raccordement (30) pour l'assemblage par engagement positif de l'unité fonctionnelle (50) et **en ce que** le levier (20) sur lequel est disposé l'entraîneur (21) est en liaison active avec la tige (12) mobile dans le sens axial pour actionner l'outil (80) disposé sur l'unité fonctionnelle (50).

17. Dispositif selon la revendication 16, **caractérisé en ce que** le mouvement dans le sens axial de la tige (12) disposée dans la douille de guidage (27) de l'élément d'assemblage (25) est limité par une partie de tête (13) disposée dessus et coopérant avec l'entraîneur (21) du levier (20) et retenue par une butée (26') prévue dans la douille de guidage (27) et l'alésage traversant (26).

18. Dispositif selon la revendication 1, **caractérisé par** l'utilisation du boîtier (10) conçu pour recevoir un entraînement (6) à actionnement électromoteur, hydraulique ou pneumatique, avec l'élément d'assemblage (25) disposé dessus, comme première unité de construction et l'unité fonctionnelle (50) munie de l'outil (80) comme seconde unité de construction, les deux unités de construction étant en liaison active entre elles par assemblage axial et par un mouvement de rotation l'une par rapport à l'autre autour de l'axe longitudinal commun et la tige (12) avec le goujon coulissant (60) pour l'actionnement de l'outil (80).

19. Dispositif selon la revendication 1, **caractérisé par** l'utilisation du boîtier (10) conçu pour recevoir un entraînement (6) pouvant être actionné par une batterie (6') avec l'élément d'assemblage (25) disposé dessus, comme première unité de construction et l'unité fonctionnelle (50) munie de l'outil (80) comme seconde unité de construction, les deux unités de construction étant en liaison active entre elles par assemblage axial et par un mouvement de rotation l'une par rapport à l'autre autour de l'axe longitudinal commun et la tige (12) avec le goujon coulissant (60) pour l'actionnement de l'outil (80).

20. Dispositif selon la revendication 1, **caractérisé par** l'utilisation du boîtier (10) muni d'au moins un levier (20) à actionnement manuel, avec l'élément d'assemblage (25), comme première unité de construction, et l'unité fonctionnelle (50) munie de l'outil (80) comme seconde unité de construction, les deux unités de construction étant en liaison active entre elles par assemblage axial et par un mouvement de rotation l'une par rapport à l'autre autour de l'axe longitudinal commun et la tige (12) avec le goujon coulissant (60) pour l'actionnement de l'outil (80).

21. Dispositif selon la revendication 1, **caractérisé par** l'utilisation du boîtier (10) muni de deux parties de boîtier (40, 40') pouvant être rapprochées l'une de l'autre, avec l'élément d'assemblage (25) disposé dessus, comme première unité de construction et l'unité fonctionnelle (50) munie de l'outil (80) comme seconde unité de construction, les deux unités de construction étant en liaison active entre elles par assemblage axial et par un mouvement de rotation l'une par rapport à l'autre autour de l'axe longitudinal commun et la tige (12) avec le goujon coulissant (60) pour l'actionnement de l'outil (80).

22. Dispositif selon la revendication 1, **caractérisé par** l'utilisation du boîtier (10), avec l'élément d'assemblage (25) disposé dessus, comme première unité de construction et avec l'unité fonctionnelle (50) munie de la pièce intercalaire (70) vissé ainsi que de l'outil (80) comme deuxième unité de construction, les deux unités de construction étant en liaison active entre elles par assemblage axial et par un mouvement de rotation l'une par rapport à l'autre autour de l'axe longitudinal commun et la tige (12) avec le goujon coulissant (60) pour l'actionnement de l'outil (80).

23. Dispositif selon la revendication 1, **caractérisé par** l'utilisation du boîtier (10), avec l'élément d'assemblage (25) et un élément intermédiaire (70) disposé dessus en engagement positif et muni d'un filetage extérieur (66), comme première unité de construction et l'unité fonctionnelle (50) munie d'un filetage intérieur (52') ainsi que de l'outil (80) comme deuxième unité de construction, les deux unités de construction étant en liaison active entre elles par l'assemblage vissé et la tige (12) et le goujon coulissant (60) pour l'actionnement de l'outil (80) entre eux.

24. Dispositif selon la revendication 1, **caractérisé par** l'utilisation de l'unité fonctionnelle (50) munie d'un filetage intérieur (52') comme première unité de construction et du boîtier (10), avec l'élément d'assemblage (25) et la pièce intercalaire (70) retenue dessus en engagement positif et muni du filetage extérieur (66), comme deuxième unité de construction les deux unités de construction étant en liaison active entre elles par l'assemblage vissé et la tige (12) et le goujon coulissant (60) pour l'actionnement de l'outil (80) entre eux.
